# FASCICULE DE BREVET EUROPEEN

(11) **EP 3 829 311 B1**
(45) Date de publication et mention de la délivrance du brevet: **11.03.2026**
(21) Numéro de dépôt: 19755301.9
(22) Date de dépôt: 02.08.2019
(51) Int. Cl.: A01N 59/00, A01N 37/36, A01N 59/06, A01N 59/08, A01N 59/16

(54) **CITRATES PERHYDRATES ET LEURS UTILISATIONS**
CITRATPERHYDRATE UND DEREN VERWENDUNGEN
CITRATE PERHYDRATES AND USES THEREOF

(30) Priorité: 03.08.2018 FR 1857317
(43) Date de publication de la demande: 09.06.2021
(60) Demande divisionnaire: 21200583.9
(73) Titulaire: Biorem Engineering SA, 1920 Martigny (CH)
(72) Inventeur: LAKAYE, Frédéric, 1911 Ovronnaz (CH); GAUME, Alain, 1680 Romont (CH); GINDRO, Katia, 1122 Romanel-sur-Morges (CH); SCHNEE, Sylvain, 01710 Thoiry (FR); HARDY, Wendy, 4250 Geer (BE); NOËL, Marc, 1325 Chaumont-Gistoux (BE); SEMER, Maurice, 4577 Modave (BE)
(74) Mandataire: Gevers & Orès
(86) Numéro de dépôt international: PCT/EP2019/070941
(87) Numéro de publication internationale: WO 2020/025816

(56) Documents cités:
- WO-A1-95/28840
- FR-A1- 2 219 150
- US-A- 3 647 869
- US-A- 4 655 975

## Description

L'invention concerne des citrates perhydrates, ainsi que les utilisations de citrates perhydrates, notamment en tant que biocides, en particulier pesticides, plus particulièrement phytopharmaceutiques.

L'invention concerne également un procédé de préparation de ces nouvelles molécules organiques biobasées, lesquelles se présentent sous forme d'une poudre cristalline stable, soluble et qui contiennent à pH physiologique une nouvelle forme solide de peroxyde d'hydrogène.

Les biocides désignent une large famille de substances chimiques qui regroupe les pesticides, en particulier les produits phytopharmaceutiques, et les antimicrobiens à usage médical, vétérinaire, domestique ou industriel, ainsi que les désinfectants des fluides et des surfaces, en particulier de l'eau, de l'air, des sols, des piscines, des surfaces de travail, des toilettes, etc.

Les antimicrobiens et les désinfectants peuvent réduire au minimum le risque d'une infection des humains ou des animaux. Puisque certaines bactéries, moisissures, levures et virus peuvent conduire à des maladies graves, la désinfection est un élément primordial de la vie quotidienne, en particulier dans le secteur médical et dans les foyers. L'importance de la désinfection pour le bien-être des humains est souvent sous-estimée. Au cours des siècles précédents, plus de personnes sont mortes à la suite des grandes épidémies (peste, choléra, variole ou grippe) que celles qui ont été tuées pendant les guerres. Jusqu'au début du XXe siècle, les infections bactériennes sévères étaient souvent mortelles même dans les pays industrialisés. Dans les pays les moins favorisés, les maladies infectieuses, qui proviennent pour la plupart de situations d'hygiène inadéquates, font aujourd'hui encore l'objet de nombreux décès. Il existe donc une forte demande d'antimicrobiens et de désinfectants efficaces et peu coûteux, en particulier dans le secteur médical.

Cette demande existe également dans le secteur agraire, pour les pesticides, en particulier pour les substances fongicides. En effet, les pesticides tels que les fongicides, les insecticides et les herbicides sont des agents auxiliaires importants pour l'agriculture afin de protéger et d'augmenter les rendements des cultures. Le personnel agricole travaille à optimiser la production en maximisant les conditions de croissance tout en minimisant les attaques contre les graines, les semis, les plantes et les fruits par les nuisibles. De tels nuisibles incluent les bactéries, les champignons, etc. Une attention considérable a été accordée aux composés antimicrobiens qui attaquent les bactéries et les champignons sur les semences, les semis, les plantes en croissance et les fruits. L'utilisation de fongicides dans l'agriculture est rendue nécessaire par les pertes causées par une grande variété de microorganismes pathogènes pour les plantes. Pour être économique, les coûts de lutte contre les maladies des plantes par l'application de bactéricides et de fongicides doivent être compensés par des gains potentiels bien supérieurs. Des tonnages importants de fongicides sont nécessaires dans la culture des pommes, des poires, des bananes, des céréales, du cacao, du café, du coton, des pommes de terre, du tabac, des raisins de table et de cuve, et d'autres fruits et légumes communs tels que le céleri, les poireaux, les oignons, la laitue, les pommes de terre, l'ail, les échalotes, les poivrons, les haricots, les tomates, les amandes, les arachides et bien d'autres.

En particulier, selon les chiffres de 2016, le marché mondial des fongicides phytosanitaires est estimé à plus de 9,7 milliards d'euros, notamment 3,9 milliards d'euros en Europe et 3,6 milliards d'euros en Amérique.

Au sein de ce marché, il existe une forte demande de fongicides dans le secteur du vin. Par exemple, en France, la consommation totale de produits fongicides pour le traitement des vignes est estimée à 400M€ pour une surface totale de 792.000,00 ha.

Les biocides conventionnels sont souvent toxiques, carcinogènes, mutagènes, tératogènes, coûteux et/ou inefficaces. De plus, pour obtenir un effet désinfectant puissant, des substances chimiques hautement persistantes ont été utilisées par le passé comme désinfectants afin d'obtenir une protection efficace et durable contre les microorganismes. Mais cette persistance conduit à des problèmes environnementaux considérables. En effet, les biocides hautement persistants s'accumulent dans les eaux souterraines et/ou dans la chaîne alimentaire et entraînent des problèmes écologiques et sanitaires majeurs. Par exemple, des problèmes écologiques peuvent survenir lorsque des biocides en forte concentration atteignent les stations d'épuration biologiques. Dans le cas de concentrations élevées en biocides, les micro-organismes qui y sont nécessaires sont affectés dans leur croissance, ce qui peut conduire à une défaillance partielle ou complète de l'installation de traitement des eaux usées. De plus, des composites persistants peuvent s'accumuler dans les boues d'épuration.

Afin de surmonter les inconvénients sanitaires et écologiques de ces biocides persistants conventionnels, l'utilisation de substances moins dangereuses a été envisagée dans le passé, en particulier l'utilisation de substances naturelles susceptibles de présenter des propriétés désinfectantes. Cependant, leur meilleure compatibilité environnementale a été obtenue au détriment de leur efficacité et de leur capacité de protection contre les microorganismes. En raison de cet inconvénient, on a accordé moins d'importance à ces désinfectants écologiquement compatibles et l'utilisation de composés dangereux pour l'environnement prévaut.

L'acide peracétique est un agent oxydant puissant qui est connu pour avoir des propriétés virucides, bactéricides, fongicides et algicides. L'acide peracétique a été breveté dans les années 50 pour le traitement des tissus végétaux notamment destinés à être transformés, en particulier pour le traitement des fruits et légumes, afin de réduire la détérioration de ces derniers par des bactéries et des champignons (brevet US 2 522 640). De nos jours, l'acide peracétique est couramment utilisé au cours de la transformation et de la manipulation des aliments comme désinfectant pour les surfaces en contact avec les aliments, mais également pour les fruits, les légumes, la viande et les œufs. Dans la production de fruits et de légumes, des solutions aqueuses peracétiques ont été suggérées pour contrôler les organismes pathogènes sur les plantes en croissance. Cependant, l'un des problèmes majeurs associé aux solutions aqueuses liquides d'acide peracétique est que ces solutions sont corrosives, fortement acides, très réactives, et fortement odorantes, ce qui rend leur utilisation mal aisée et dangereuse, à la fois pour les utilisateurs et pour les plantes traitées.

Il a maintenant été mis au point des nouveaux composés biobasés, zéro résidu, de type citrate perhydrate, faisant l'objet de la présente demande, permettant de libérer des agents actifs, à savoir du peroxyde d'hydrogène ainsi que des citrates, lesquels peuvent agir en synergie, qui ont une efficacité équivalente aux biocides de référence tout en étant tous classés GRAS (« Generally Recognized As Safe ») par la Food and Drug Administration. En effet, les produits de dégradation des citrates perhydrates de l'invention sont des additifs alimentaires tels que, par exemple, les citrates de sodium (E331), de potassium, de zinc, etc.

De plus, les composés de l'invention peuvent être sous forme de poudre, que l'on peut facilement synthétiser selon un procédé économique, conditionner, transporter et manipuler. Ces poudres sont stables physiquement et chimiquement, c'est-à-dire qu'il n'y a pas de modification significative, sous l'effet du temps (par exemple après un an à température ambiante, soit à une température comprise de 20 à 25°C), de leur état physique et de leur composition chimique, notamment de leur concentration en oxygène actif. Elles sont en outre sans odeur, très solubles dans l'eau, et les solutions aqueuses correspondantes ont une large gamme de pH possible, située entre 4 et 8. En effet, le pH obtenu en mettant en solution d'autres composés contenant du peroxyde d'hydrogène (par exemple un pH de 11 pour le percarbonate de sodium) peut ne pas être compatible avec le traitement des plantes. Ces composés sont faciles à mettre en œuvre, notamment parce qu'ils permettent de s'affranchir de l'utilisation de solutions concentrées de peroxyde d'hydrogène, bien connues pour être oxydantes, irritantes et susceptibles de provoquer un incendie ou une explosion.

En outre, ces composés permettent une application aisée et une manipulation sûre. De plus, ces solutions forment au séchage un biofilm protecteur antimicrobien rendant la surface traitée plus résistante, notamment aux contaminations bactériennes et/ou fongiques.

De façon tout-à-fait surprenante, les citrates perhydrates de l'invention sont stables physiquement et chimiquement. A contrario, les solutions concentrées d'acide citrique et de peroxyde d'hydrogène, mélangés, ne cristallisent pas sous forme de perhydrates et ne sont pas stables chimiquement, notamment en ce qui concerne la concentration en oxygène actif. Les composés de l'invention permettent donc de stabiliser le peroxyde d'hydrogène concentré solide, sous forme de nouvelles molécules de citrates perhydratés.

Par exemple, une solution contenant 37,5% en peroxyde d'hydrogène et 25% d'acide citrique perd au bout de 20 jours en moyenne 34,4% de sa teneur en peroxyde d'hydrogène. Cette instabilité est la conséquence de l'oxydation de l'acide citrique, par le peroxyde d'hydrogène, en acétone dicarboxylique, qui est à son tour oxydé en formaldéhyde, acide formique et dioxyde de carbone. Une seconde époxydation de l'acide citrique a également lieu et engendre la formation d'acide percitrique (exemple 7).

Ainsi, selon un premier aspect, l'invention concerne un citrate perhydrate de métal alcalin, le citrate perhydrate est un citrate disodique perhydrate, notamment un citrate disodique monoperhydrate anhydre.

Selon un mode de réalisation, le citrate perhydrate de métal alcalin est sous la forme d'un cristal constitué de citrate de métal alcalin, de peroxyde d'hydrogène, et éventuellement d'eau.

Selon un mode de réalisation, ledit citrate perhydrate est sous la forme d'un hydrate, en particulier un monohydrate ou un dihydrate.

Selon un mode de réalisation, ledit citrate perhydrate est un citrate monoperhydrate ou un citrate diperhydrate, en particulier un citrate monoperhydrate.

Selon un mode de réalisation particulier, ledit citrate perhydrate est un citrate disodique monoperhydrate co-cristallisé avec de l'urée perhydrate.

Selon un mode de réalisation particulier, ledit citrate perhydrate est un citrate perhydrate disodique non hydraté.

Les hydrates de l'invention sont de façon avantageuse aisément solubles dans l'eau, notamment à 25°C, leur solubilité étant en particulier supérieure ou égale à 850 g/l à 25°C.

Selon un mode de réalisation, ledit citrate perhydrate est sous la forme d'un cristal, en particulier d'un hydrate cristallin. Selon un mode de réalisation, ledit citrate perhydrate est utilisé en l'absence de composés N-acyle destinés à acyler le peroxyde d'hydrogène.

Selon un mode de réalisation, ledit citrate perhydrate est utilisé en présence d'urée perhydrate.

Selon un mode de réalisation particulier, ledit citrate perhydrate est co-cristallisé avec de l'urée perhydrate.

Selon un mode de réalisation avantageux, ledit citrate perhydrate est utilisé en présence d'urée perhydrate sous forme de cristal, ces deux cristaux étant notamment sous la forme de co-cristaux.

Selon un mode de réalisation avantageux, ledit citrate perhydrate est un citrate disodique perhydrate co-cristallisé avec de l'urée perhydrate.

En particulier, l'urée perhydrate est sous la forme d'un co-cristal urée-peroxyde d'hydrogène. L'utilisation conjointe d'urée perhydrate a pour avantage d'apporter une source d'azote tout en augmentant la concentration en peroxyde d'hydrogène. Selon un mode de réalisation, ledit citrate perhydrate est utilisé en présence d'eau.

Selon un mode de réalisation, ledit citrate perhydrate est utilisé en présence d'au moins un composé additionnel choisi parmi les agents anti-agglomérants, les tensioactifs, en particulier les biosurfactants, les agents mouillants, les agents anti-mousse, les anti-dérives, les épaississants, les agents moussants, les engrais, les produits phytopharmaceutiques, les stabilisants et leurs mélanges, le composé additionnel étant notamment choisi parmi les glycolipides, le pyrophosphate de disodium, le cocoyl iséthionate de sodium, l'heptaméthyltrisiloxane, le silicate de disodium, les oxydes ou peroxydes de zinc et le dioxyde de silicium.

Selon un mode de réalisation particulier, ledit citrate perhydrate est utilisé en mélange avec de l'eau et éventuellement au moins un composé additionnel choisi parmi les agents anti-agglomérants, les tensioactifs, en particulier les biosurfactants, les agents mouillants, les agents anti-mousse, les anti-dérives, les épaississants, les agents moussants, les agents de solidification, les engrais, les produits phytopharmaceutiques, les stabilisants et leurs mélanges, le composé additionnel étant notamment choisi parmi les glycolipides, le pyrophosphate de disodium, le cocoyl iséthionate de sodium, l'heptaméthyltrisiloxane, le silicate de disodium, les oxydes ou peroxydes de zinc et le dioxyde de silicium, le pourcentage en poids dudit citrate perhydrate de métal alcalin, alcalino-terreux, de transition ou pauvre par rapport au poids total dudit mélange étant notamment de 0,05 à 5%.

Plus particulièrement, le pourcentage en poids dudit citrate perhydrate par rapport au poids total dudit mélange est de 0,1 à 2, 2,5, 3, 3,5, 4 ou 4,5%.

De tels composés additionnels sont bien connus de l'homme du métier. Ils peuvent être préformulés en mélange avec du citrate perhydrate, ou ajoutés au mélange obtenu par la mise en contact du citrate perhydrate avec de l'eau.

Selon un mode de réalisation particulier, ledit au moins un composé additionnel est choisi dans le groupe comprenant le citrate d'urée, un co-cristal d'acide citrique et d'urée, éventuellement perhydraté, et de l'urée perhydrate, en particulier sous forme de cristal. Les composés de ce groupe permettent notamment l'ajustement du pH et/ou l'ajustement du taux de peroxyde d'hydrogène et/ou l'ajustement de l'apport d'azote lors de l'utilisation des citrates perhydrates de l'invention.

Lorsque le composé de l'invention est utilisé en présence d'un composé comprenant du magnésium, ces composés sont en sus utilisés en tant qu'engrais, notamment engrais foliaire, ou en tant qu'amendement.

Les stabilisants sont notamment des stabilisants de peroxydes. Ces stabilisants sont bien connus de l'homme du métier, en particulier en tant que stabilisants du peroxyde d'hydrogène.

L'ajout d'un agent moussant peut permettre d'obtenir un mélange selon l'invention sous la forme d'une mousse. Les mousses peuvent être utilisées sur les surfaces.

L'ajout d'un épaississant peut permettre d'obtenir un mélange selon l'invention sous la forme d'un gel. Les gels peuvent être utilisés sur la peau chez l'homme ou l'animal, en particulier les mains.

L'ajout d'un agent de solidification peut permettre d'obtenir un mélange selon l'invention sous la forme d'un solide. Les solides peuvent être utilisés pour traiter l'eau, en particulier dans les piscines et les toilettes.

Selon un mode de réalisation avantageux, ledit composé additionnel est choisi parmi les glycolipides, en particulier les rhamnolipides.

Ces rhamnolipides peuvent être obtenus selon les techniques bien connues de l'homme du métier, notamment en cultivant des bactéries du genre *Pseudomonas* en présence de mélasse.

Au sein des compositions de la présente invention, les glycolipides, en particulier les rhamnolipides, possèdent d'avantageuses propriétés élicitrices et biosurfactantes.

Selon un autre aspect, l'invention concerne l'utilisation d'un citrate perhydrate selon l'invention en tant que biocide.Selon un mode de réalisation, l'utilisation telle que définie ci-dessus est une utilisation pour inhiber la croissance d'un pathogène sur ou dans une plante.

Ainsi, la présente invention concerne également l'utilisation d'un composé tel que décrit précédemment ou d'une composition telle que décrite précédemment en tant qu'inhibiteur de la croissance d'un pathogène sur ou dans une plante.

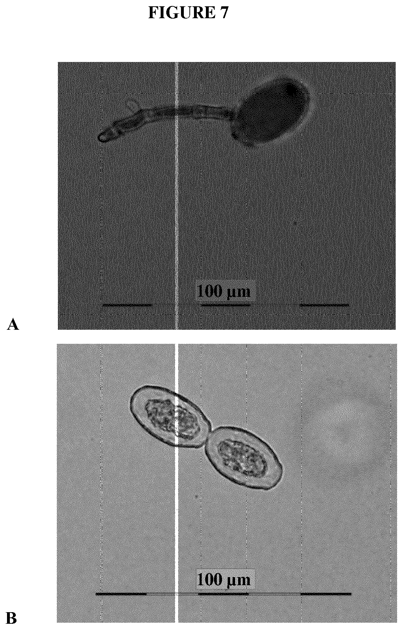

Sans vouloir se limiter à une quelconque théorie, ledit citrate perhydrate provoque un stress oxydatif, ionique et osmotique au pathogène visé et engendre la constriction de son contenu cellulaire dans le cas, par exemple, du pathogène *Erysiphe necator,* comme illustré à la figure 7.

Selon un mode de réalisation, l'utilisation telle que définie ci-dessus est une utilisation pour prévenir la croissance d'un pathogène sur ou dans une plante.

La présente invention concerne alors l'utilisation d'un composé tel que décrit précédemment ou d'une composition telle que décrite précédemment dans la prévention de la croissance d'un pathogène sur ou dans une plante.

Sans vouloir se limiter à une quelconque théorie, la prévention se fait grâce aux propriétés collantes attribuées aux citrates et/ou aux éventuels agents collants additionnés, ce qui a pour conséquence de former une barrière protectrice autour de la surface traitée, empêchant, par exemple, la propagation de spores.

En particulier, ledit citrate perhydrate est appliqué à la surface de la plante, en particulier à hauteur de 25 à 1000 ng.dm⁻².

Selon un mode de réalisation, ledit citrate perhydrate, éventuellement utilisé en présence d'eau, est appliqué à la surface de la plante par pulvérisation, vaporisation, trempage, badigeonnage, fumigation ou pulvérisation électrostatique, de préférence par pulvérisation, en particulier par pulvérisation foliaire.

Selon un mode de réalisation, ledit citrate perhydrate, éventuellement utilisé en l'absence d'eau, est appliqué sur les racines de la plante, ou dans le sol au contact avec les racines de la plante. Selon un mode de réalisation, le pathogène est sélectionnée parmi les virus, les bactéries, les champignons et les pseudo-champignons.

Selon un mode de réalisation particulier, le pathogène un champignon ou un pseudo-champignon sélectionné parmi *Albugo spp., Alternaria spp., Armillaria spp., Aspergillus spp., Athelia spp., Bipolaris spp., Botryosphaeria spp., Botryotinia spp., Botrytis spp., Bremia spp., Candida spp., Capnodium spp., Ceratobasidium spp., Ceratocystis spp., Cercospora spp., Choanephora spp., Claviceps spp., Corynespora spp., Cronartium spp., Cryphonectria spp., Cylindrocladium spp., Cytospora spp., Diaporthe spp., Diplodia spp., Dreschlera spp., Elsinoe spp., Erexohilum spp., Erysiphe spp., Eutypa spp., Exobasidium spp., Fusarium spp., Gaeumannomyces spp., Gliocladium spp., Gymnosporangium spp., Heterobasidium spp., Hypoxylon spp., Kutilakesa spp., Lophiodermium spp., Magnaporthe spp., Melampsora spp., Monilinia spp., Mycosphaerella spp., Myrothecia spp., Nectriella spp., Nematospora spp., Oïdium spp., Olpidium spp., Ophiostoma spp., Penicillium spp., Peronospora spp., Phakospora spp., Phoma spp., Phomopsis spp., Phragmidium spp., Phyllactinia spp., Physoderma spp., Phytophthora spp., Plasmodiophora spp., Plasmopara spp., Pseudoperonospora spp., Puccinia spp., Pythium spp., Rhizoctonia spp., Rhizopus spp., Rhytisma spp., Sclerotinia spp., Sclerotium spp., Spongospora spp., Synchytrium spp., Taphrina spp., Thanatephorus spp., Thielaviopsis spp., Tilletia spp., Uncinula spp., Urocystis spp., Ustilago spp., Valsa spp., Venturia spp., Verticillium spp., Xylaria spp, Fomitiporia spp., Stereum spp., Phaeoacremonium spp.* et *Phaeomoniella spp.*

Selon un mode de réalisation encore plus particulier, le pathogène un champignon ou un pseudo-champignon sélectionné parmi *Plasmopara spp., Erysiphe spp., Botrytis spp., Aspergillus spp.* et *Candida spp.*

Selon un mode de réalisation particulier, le pathogène est une bactérie sélectionnée parmi les bactéries du genre *Pseudomonas, Escherichia, Staphylococcus, Enterococcus,* et *Legionella.*

Selon un mode de réalisation particulier, le pathogène est un ou plusieurs microorganismes sélectionnés parmi *Candidatus phytoplasma, Fomitiporia punctata, F. mediteranea, Stereum hirsutum, Phaeoacremonium aleophilium, Phaeomoniella chlamydospora, Botryosphaeria obtusa, Botryosphaeria dothidea parva et stevensii,* et *Eutypa lata.*

Selon un mode de réalisation, la plante est choisie parmi les plantes produisant des fruits, les plantes produisant des légumes, les plantes ornementales, le gazon et les céréales.

Par plante ornementale, on entend notamment une plante cultivée pour ses qualités d'agrément.

Par gazon, on entend notamment l'ensemble des herbes fines, notamment des graminées, constituant ou compris dans les pelouses.

Selon un mode de réalisation particulier, la plante produit des fruits choisis parmi pomme, abricot, banane, mûre, myrtille, cerise, canneberge, groseille, raisin de table, raisin de cuve (la plante étant en particulier la vigne), grenade, groseille, melon, citron, mandarine, melon, orange, pêche, poires, ananas, prune, framboise, fraise, tomates, pastèque, pamplemousse, poivre, olives, citron vert, amandes, noix, noix du Brésil, noix de cajou, châtaignes, noisettes, noix de macadamia, noix de pécan et pistaches.

Selon un mode de réalisation particulier, la plante produit des légumes choisis parmi artichaut, haricots, betterave, brocoli, chou, carotte, chou-fleur, céleri, chicorée, ciboulette, cresson, concombre, chou frisé, aubergine, chou-rave, laitue, oignon, poivron, panais, persil, pois, pomme de terre, potiron, radis, échalote, soja, épinards, navets et cacahouètes.

Selon un mode de réalisation particulier, la plante est une céréale, notamment sous la forme de chaumes.

Selon un mode de réalisation particulier, la plante est une céréale choisie parmi amarante, orge, sarrasin, fonio, kamut (blé de Khorasan), millet, avoine, quinoa, riz, seigle, sorgho, épeautre, triticale, blé, ou colza.

Selon un mode de réalisation, la présente invention concerne également l'utilisation d'un composé tel que décrit précédemment ou d'une composition telle que décrite précédemment en tant qu'élément de biocontrôle.

Selon un mode de réalisation, la présente invention concerne l'utilisation d'un citrate perhydrate pour désinfecter un fluide ou une surface, en particulier l'eau, l'air, les sols, les piscines, les surfaces de travail, les toilettes.

Selon un mode de réalisation, la présente invention concerne l'utilisation d'un citrate perhydrate pour désinfecter un fluide, en particulier l'eau ou l'air, en réduisant notamment le nombre de cellules bactériennes viables, les cellules bactériennes étant en particulier du genre *Legionella,* plus particulièrement *L. pneumophila.*

Ainsi, ledit citrate perhydrate peut être utilisé en tant que désinfectant d'un fluide ou d'une surface.

Des installations telles que les spas, les piscines, les tours de refroidissement, les installations de production agro-alimentaire, les milieux hospitaliers, sont susceptibles d'être désinfectées par un composé tel que décrit précédemment ou une composition telle que décrite précédemment.

Selon un autre mode de réalisation, la présente invention concerne l'utilisation d'un citrate perhydrate pour désinfecter une surface, notamment en collectivités, en milieu hospitalier ou dans les installations de production agroalimentaire, en particulier les sols, les surfaces de travail, les toilettes.

Selon un mode de réalisation, la présente invention concerne l'utilisation d'un citrate perhydrate pour désinfecter une surface, en réduisant notamment le nombre de cellules bactériennes viables ou de champignons, les cellules bactériennes étant en particulier sélectionnées parmi *Pseudomonas aeruginosa, Escherichia coli, Staphylococcus aureaus, Enterococcus hirae,* et les champignons étant en particulier sélectionnés parmi *Candida albicans* et *Aspergillus niger.*

Selon un mode de réalisation, la présente invention concerne l'utilisation d'un citrate perhydrate telle que définie précédemment, en tant qu'éliciteur.

Selon un mode de réalisation, la présente invention concerne un citrate perhydrate pour son utilisation non thérapeutique en tant qu'antimicrobien chez l'homme ou l'animal.

Il est à noter que tous les modes de réalisation mentionnés ci-dessus à propos du citrate perhydrate s'appliquent également ici, seuls ou en combinaison.

Selon un mode de réalisation particulier, le citrate perhydrate est utilisé en médecine dentaire.

Selon un autre mode de réalisation particulier, le citrate perhydrate est utilisé chez l'animal, notamment pour le traitement des mamelles, en particulier des vaches, ou des pieds, en particulier du cheval.

Selon un autre aspect, la présente invention concerne une méthode de blanchiment des dents par mise en contact des dents avec un citrate perhydrate.

Selon un autre aspect, la présente invention un citrate perhydrate tel que décrit précédemment, pour son utilisation en médecine dentaire, notamment dans le blanchiment des dents.

Il est à noter que tous les modes de réalisation mentionnés ci-dessus à propos du citrate perhydrate s'appliquent également ici, seuls ou en combinaison.

Selon un mode de réalisation, la présente invention concerne un citrate perhydrate pour son utilisation en tant que bactéricide ou bactériostatique, notamment vis-à-vis de bactéries sélectionnées parmi *Pseudomonas aeruginosa, Escherichia coli, Staphylococcus aureaus, Enterococcus hirae.*

Selon un mode de réalisation, ladite utilisation se fait par voie topique.

Selon un autre aspect, la présente invention concerne une composition pharmaceutique constitué de ou comprenant un citrate perhydrate et un excipient pharmaceutiquement acceptable.

Il est à noter que tous les modes de réalisation mentionnés ci-dessus à propos du citrate perhydrate s'appliquent également ici, seuls ou en combinaison.

Selon l'invention, le citrate perhydrate de métal alcalin est un citrate disodique monoperhydrate.

Selon un mode de réalisation, le citrate perhydrate de métal alcalin est sous la forme d'un cristal constitué d'acide citrique et/ou de citrate de métal alcalin, de peroxyde d'hydrogène, et éventuellement d'eau.

Selon un mode de réalisation, le citrate perhydrate de métal alcalin est sous la forme d'un hydrate.

Les citrates perhydrates de l'invention peuvent être des cristaux, uniques. En effet, la signature obtenue par mesure de diffraction aux rayons X leur est propre. Ces cristaux se distinguent en particulier par le nombre et la nature des métaux alcalins, par le nombre d'adduits de molécules de peroxyde d'hydrogène et par le nombre d'adduits de molécules d'eau.

Selon un mode de réalisation, le citrate perhydrate de métal alcalin est un citrate disodique perhydrate présentant un spectre de diffraction des rayons X avec les raies caractéristiques aux angles 2Θ (en °) suivantes, notamment les raies caractéristiques dont l'intensité relative est supérieure ou égale à 15 :

| **Pics** | **2Θ** | **Intensité relative** | **Index hkl** |
|---|---|---|---|
| 1 | 7,10442 | 2,42 | 0 1 1 |
| 2 | 6,53808 | 6,93 | 1 1 1 |
| 3 | 6,19763 | 11,5 | 0 2 0 |
| 4 | 5,03008 | 18,94 | 2 2 0 |
| 5 | 4,83206 | 0,76 | 1 2 1 |
| 6 | 4,45527 | 10,07 | 3 1 1 |
| 7 | 4,34762 | 32,9 | 2 2 1 |
| 8 | 4,29193 | 11,19 | 4 0 0 |
| 9 | 4,21333 | 17,21 | 3 2 0 |
| 10 | 3,97003 | 19,47 | 1 1 2 |
| 11 | 3,85293 | 13,47 | 4 0 1 et 2 0 2 |
| 12 | 3,78756 | 19,04 | 3 2 1 |
| 13 | 3,67804 | 12,75 | 4 1 1 |
| 14 | 3,53769 | 100 | 4 2 0 |
| 15 | 3,47303 | 5,61 | 1 2 2 |
| 16 | 3,42705 | 7,44 | 2 3 1 |
| 17 | 3,32368 | 6,66 | 3 1 2 |
| 18 | 3,2801 | 32,86 | 2 2 2 |
| 19 | 3,13095 | 31,11 | 3 3 1 |
| 20 | 3,09689 | 19,5 | 5 1 1 |

Selon un mode de réalisation avantageux, ledit citrate perhydrate est un citrate disodique perhydrate co-cristallisé avec de l'urée perhydrate, notamment un citrate disodique perhydrate co-cristallisé avec de l'urée perhydrate.

Selon un autre aspect, l'invention concerne également une composition constituée de ou comprenant un citrate perhydrate tel que défini précédemmentet de l'urée perhydrate.

En particulier, l'urée perhydrate est sous la forme d'un co-cristal urée-peroxyde d'hydrogène.

Selon un mode de réalisation avantageux, l'invention concerne une composition comprenant des cristaux de citrate disodique perhydrate et des cristaux d'urée perhydrate, notamment sous la forme d'un co-cristal urée-peroxyde d'hydrogène. Les cristaux de citrate disodique perhydrate et ceux d'urée perhydrate sont en particulier co-cristallisés.

Selon un autre aspect, l'invention concerne un procédé de préparation d'un citrate perhydrate de métal alcalin tel que défini précédemment, ledit procédé comprenant :
(i) une étape de mise en contact d'un citrate disodique avec du peroxyde d'hydrogène, pour obtenir ledit citrate perhydrate de métal alcalin.

De façon préférée, les quantités de citrate disodique et de peroxyde d'hydrogène sont stœchiométriques, selon la composition du citrate perhydrate de métal alcalin désiré.

Les quantités stœchiométriques sont par exemple mises en œuvre à l'aide d'un doseur gravimétrique.

Selon un mode de réalisation avantageux, le citrate disodique est sous forme solide, en particulier anhydre, et le peroxyde d'hydrogène est sous la forme d'une solution aqueuse de peroxyde d'hydrogène à une concentration de 30 à 80% en poids.

L'étape (i) est notamment réalisée à une température comprise de 30 à 90°C, en particulier de 40 à 80°C, plus particulièrement de 50 à 70 °C, notamment de 55 à 65°C.

Selon un mode de réalisation très avantageux, la mise en contact se fait au moyen d'une extrudeuse, par exemple une extrudeuse bivis.

Selon un autre mode de réalisation avantageux, la composition obtenue à l'étape (i) est admise dans un alcool ou une solution alcoolique, ledit alcool étant notamment un alcool en C1 à C5. L'utilisation de cet alcool peut notamment permettre d'obtenir la précipitation du citrate perhydrate désiré, lequel peut être isolé par des techniques bien connues de séparation liquide/solide.

Selon un mode de réalisation très avantageux, la mise en contact se fait par mélange d'une solution aqueuse contenant un citrate disodique et du peroxyde d'hydrogène ; et de l'alcool ou la solution alcoolique, le ratio volumique de l'alcool ou de la solution alcoolique par rapport à la solution aqueuse étant notamment compris de 3,7 : 1 à 8 : 1.

Selon un mode de réalisation, l'étape (i) est précédée d'une étape de neutralisation d'un acide citrique par un hydroxyde, un carbonate ou un citrate de sodium, afin d'obtenir le citrate disodique tel que mentionné dans l'étape (i).

Cette étape de neutralisation peut être totale ou partielle.

Selon un autre mode de réalisation, une étape de neutralisation d'un acide citrique par un hydroxyde, un carbonate ou un citrate de sodium, afin d'obtenir le citrate disodique tel que mentionné dans l'étape (i), est réalisée concomitamment à cette étape (i).

En particulier, l'invention concerne un procédé de préparation d'un citrate perhydrate de métal alcalin tel que défini précédemment, ledit procédé comprenant :
(i') une étape de mise en contact d'acide citrique ; d'un hydroxyde, carbonate ou citrate de sodium ; et de peroxyde d'hydrogène, pour obtenir ledit citrate perhydrate de métal alcalin.

### L'hydroxyde, carbonate ou citrate de sodium est notamment un citrate de sodium

De façon préférée, les quantités d'acide citrique/citrate, de métal alcalin, et de peroxyde d'hydrogène sont stœchiométriques, selon la composition du citrate perhydrate de métal alcalin désiré.

Selon un mode de réalisation particulier, l'étape (i) ou (i') telle que définie précédemment est suivie d'une étape de séchage et/ou de broyage.

Ces étapes peuvent être réalisées selon les techniques bien connues de l'homme du métier.

Selon un autre aspect, l'invention concerne un procédé de préparation d'une composition constituée de ou comprenant un citrate perhydrate selon l'invention, et de l'urée perhydrate, telle que notamment définie précédemment, ledit procédé comprenant une étape (a) de co-cristallisation par mise en contact d'un citrate disodique ; d'urée ; et de peroxyde d'hydrogène.

De façon préférée, les quantités de citrate disodique et de peroxyde d'hydrogène sont stœchiométriques, selon la composition du citrate perhydrate de métal alcalin désiré.

De façon préférée, la quantité d'urée est comprise de 1 à 5 moles d'urée par mole de sel d'acide citrique, de préférence de 2 à 4 moles d'urée par mole de sel d'acide citrique, en particulier de 3 moles d'urée par mole de sel d'acide citrique
Selon un mode de réalisation avantageux, le citrate disodique ; et l'urée sont sous forme solide, et le peroxyde d'hydrogène est sous la forme d'une solution aqueuse de peroxyde d'hydrogène à une concentration de 30 à 80% en poids.

L'étape (a) est notamment réalisée à une température comprise de 30 à 90°C, en particulier de 40 à 80°C, plus particulièrement de 50 à 70 °C, notamment de 55 à 65°C.

Selon un mode de réalisation très avantageux, la mise en contact se fait au moyen d'une extrudeuse, par exemple une extrudeuse bivis.

Selon un mode de réalisation, l'étape (a) est précédée d'une étape de neutralisation d'un acide citrique par un hydroxyde, un carbonate ou un citrate de sodium, afin d'obtenir le citrate disodique tel que mentionné dans l'étape (a).

Cette étape de neutralisation peut être totale ou partielle.

Selon un autre mode de réalisation, une étape de neutralisation d'un acide citrique par un hydroxyde, un carbonate ou un citrate de sodium, afin d'obtenir le citrate disodique tel que mentionné dans l'étape (a), est réalisée concomitamment à cette étape (a).

En particulier, l'invention concerne un procédé de préparation d'une composition constituée de ou comprenant un citrate perhydrate tel que défini précédemment, et de l'urée perhydrate, telle que notamment définie précédemment, ledit procédé comprenant :
(a') une étape de mise en contact d'acide citrique ; d'un hydroxyde, carbonate ou citrate de sodium ; d'urée ; et de peroxyde d'hydrogène, pour obtenir ledit citrate perhydrate de métal alcalin.

L'hydroxyde carbonate ou citrate de sodium est notamment un citrate de sodium.

De façon préférée, les quantités de citrate de sodium et de peroxyde d'hydrogène sont stœchiométriques, selon la composition du citrate perhydrate de métal alcalin désiré.

De façon préférée, la quantité d'urée est comprise de 1 à 5 moles d'urée par mole de sel d'acide citrique, de préférence de 2 à 4 moles d'urée par mole de sel d'acide citrique, en particulier de 3 moles d'urée par mole de sel d'acide citrique

Selon un mode de réalisation particulier, l'étape (a) ou (a') telle que définie précédemment est suivie d'une étape de séchage et/ou de broyage.

Ces étapes peuvent être réalisées selon les techniques bien connues de l'homme du métier.

### Définitions

Tel qu'on l'utilise dans la présente description, le terme « environ » se réfère à un intervalle de valeurs de ± 10 % d'une valeur spécifique. A titre d'exemple, l'expression « environ 120 mg » comprend les valeurs de 120 mg ± 10 %, soit les valeurs de 108 mg à 132 mg.

Au sens de la présente description, les pourcentages se réfèrent à des pourcentages en poids par rapport au poids total de la formulation, sauf indication contraire.

Tel qu'on l'entend ici, les plages de valeur sous forme de « x-y » ou « de x à y » ou « entre x et y » incluent les bornes x et y ainsi que les entiers compris entre ces bornes. A titre d'exemple, « 1-5 », ou « de 1 à 5 » ou « entre 1 et 5 » désignent les entiers 1, 2, 3, 4 et 5. Les modes de réalisations préférés incluent chaque entier pris individuellement dans la plage de valeur, ainsi que toute sous-combinaison de ces entiers. A titre d'exemple, les valeurs préférées pour « 1-5 » peuvent comprendre les entiers 1, 2, 3, 4, 5, 1-2, 1-3, 1-4, 1-5, 2-3, 2-4, 2-5, etc.

Par « citrate », on entend un sel d'acide citrique, c'est-à-dire qu'au moins l'un des trois groupes acides carboxyliques de l'acide citrique, en particulier deux des trois ou les trois groupes acide carboxylique de l'acide citrique, est sous forme de sel, en particulier de sel alcalin, alcalino-terreux, de transition ou pauvre.

Comme exemples de sels peuvent être cités les sels avec les métaux alcalins (sodium, potassium, lithium), avec les métaux alcalino-terreux (magnésium, calcium), avec les métaux de transition, avec des métaux pauvres (zinc), le sel d'ammonium, le sel d'urée, les sels de bases azotées (éthanolamine, diéthanolamine, triméthylamine, triéthylamine, méthylamine, propylamine, diisopropylamine, NN-diméthyl- éthanolamine, benzylamine, dicyclohexylamine, N-benzyl-p-phénéthylamine, NN'-dibenzyléthylènediamine, diphénylènediamine, benzhydrylamine, quinine, chloline, arginine, lysine, leucine, dibenzylamine).

Par « citrate perhydrate » (ou citrate perhydraté, ou citrate peroxyhydrate, ou citrate peroxyhydraté, ou citrate peroxosolvate, ces termes étant ici équivalents), on entend en particulier un adduit entre un citrate et du peroxyde d'hydrogène, le citrate et le peroxyde d'hydrogène étant plus particulièrement liés, au sein d'un même solide, encore plus particulièrement d'un même cristal, par des liaisons hydrogènes. Ainsi, de manière préférée, le terme « citrate perhydrate » ne désigne pas un percitrate éventuellement hydraté.

Ainsi, un citrate perhydrate est notamment de formule (I) suivante 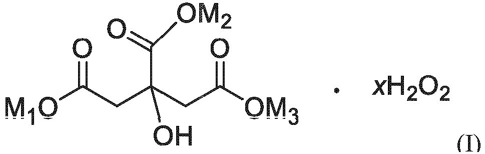 dans laquelle x est strictement supérieur à 0, x étant notamment 1 ou 2, et M₁, M₂ et M₃ représentent indépendamment les uns des autres H ou un atome ou composé susceptible de former un sel avec un groupe acide carboxylique, notamment un métal alcalin, alcalino-terreux, de transition ou pauvre, en particulier un métal alcalin, l'un au moins de M₁, M₂ et M₃ ne représentant pas H. Il est à noter que cette formule (I) peut être combinée à toute autre définition ou mode de réalisation qui précède.

Par métal pauvre, on entend notamment un élément chimique métallique situé, dans le tableau périodique, entre les métaux de transition à leur gauche et les métalloïdes à leur droite. A titre d'exemple, le métal pauvre peut être le zinc.

En particulier, seul un des groupes M₁, M₂ et M₃ représente H. Si par exemple M=Na, on parle de disodique.

En particulier, aucun des groupes M₁, M₂ et M₃ ne représente H. Si par exemple M=Na, on parle de trisodique.

En particulier, deux des groupes M₁, M₂ et M₃ représente H. Si par exemple M=Na, on parle de monosodique.

Lorsque M₁, M₂ et/ou M₃ représentent un métal alcalino-terreux, de transition ou pauvre, ce métal peut en outre être lié à un autre carboxylate, présent sur le même citrate ou sur un autre citrate de formule (I).

Par exemple, l'un de M₁, M₂ et M₃ représente un métal alcalino-terreux, les deux autres étant H. Dans ce cas, le citrate de formule (I) correspondant est notamment de formule (C₆H₇O₇)(métal alcalino-terreux)_{½}.x H₂O₂.

Par « biocide », on entend les pesticides, ainsi que les antimicrobiens à usage médical, vétérinaire, domestique ou industriel, et les désinfectants des fluides et des surfaces, en particulier de l'eau, de l'air, des sols, des piscines, des surfaces de travail, des toilettes, etc.

Par « surface », et sauf indication contraire, on entend notamment les surfaces de tissus vivants, en particulier la surface des plantes (par exemple les surfaces foliaires), et la peau (chez l'homme ou l'animal), ainsi que les surfaces inertes, notamment les surfaces inertes organiques ou inorganiques, par exemple les sols et les surfaces de travail.

Dans le cas des pesticides, la surface considérée est notamment celle d'une plante.

Dans le cas des antimicrobiens, la surface considérée est notamment celle de la peau, chez l'homme ou chez l'animal.

Dans le cas des désinfectants, la surface considérée est notamment une surface inerte.

Par « pesticide », on entend une substance chimique susceptible d'être utilisée pour lutter contre des organismes considérés comme nuisibles. C'est un terme générique qui rassemble les insecticides, les fongicides, les herbicides, les parasiticides. Ils s'attaquent respectivement aux insectes ravageurs, aux champignons, aux « mauvaises herbes » et aux vers parasites.

Par « composition phytopharmaceutique », on entend un produit phytosanitaire, c'est-à-dire tout produit destiné à :
- protéger les végétaux ou les produits végétaux contre tous les organismes nuisibles ou à prévenir leur action ;
- exercer une action sur les processus vitaux des végétaux, pour autant qu'il ne s'agisse pas de substances nutritives (par exemple, les régulateurs de croissance) ;
- assurer la conservation des produits végétaux ;
- détruire les végétaux indésirables ; et/ou
- détruire les parties de végétaux, freiner ou prévenir une croissance indésirable des végétaux.

Par « antimicrobien », on entend une substance qui tue ou ralentit la croissance des microbes tels les bactéries, les mycètes, les virus, ou les parasites, notamment chez l'homme ou l'animal.

Par « désinfectant », on entend un produit qui tue ou inactive des micro-organismes, tels les bactéries, virus et protozoaires, sur des surfaces inertes ou au sein de fluides tels que l'eau et l'air.

Par « hydrate », on entend un composé formé notamment par l'union d'un citrate perhydrate et d'eau. L'hydrate est en particulier un sel cristallisé. On parle alors d'eau de cristallisation.

Par « non hydraté », on entend un composé dépourvu d'eau de cristallisation.

Par « biosurfactant », on entend un tensioactif synthétisé par un organisme vivant.

Par « pseudo-champignons », on entend des organismes choisis notamment parmi les Oomycètes, les Hyphochytridiomycètes et les Labyrinthulomycètes.

Par « chaume », on entend un résidu de culture constitué par la partie des tiges de céréales qui reste sur le sol après la moisson.

Par « éliciteur », on entend un composé ou une composition qui déclenche les mécanismes de défense des plantes avec production de substances défensives. Il s'agit d'un stimulateur des défenses naturelles (SDN) de la plante.

Tel qu'il est utilisé ici, le terme «pharmaceutiquement acceptable» se réfère à des composés, compositions et / ou formes de dosage qui sont, dans la portée d'un jugement médical valable, adapté pour une utilisation en contact avec les cellules des humains et des animaux inférieurs sans toxicité, irritation, réponse allergique indue et similaires, et sont proportionnés à un rapport avantage/risque raisonnable.

Les termes « citrate sodique, disodique, trisodique » sont dans toute cette description, et sauf mention contraire, interchangeables avec les termes « citrate (de) sodium, disodium, trisodium », respectivement. Il en est de même pour les autres métaux.

Par « co-cristallisés », on entend notamment deux cristaux de composés différents, obtenus à partir d'une solution, notamment par évaporation de celle-ci, comprenant ces deux composés. Au moins l'un ces deux composés peut être sous la forme d'un co-cristal.

Ainsi, en particulier, des cristaux co-cristallisés forment une composition où coexistent deux cristaux de nature différente, au contraire d'un co-cristal, étant un seul et même cristal comprenant ou constitué de deux composés différents.

### FIGURES

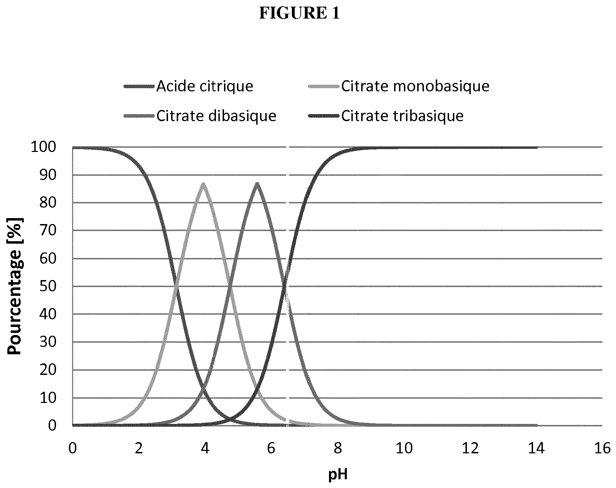

La **figure 1** représente le diagramme de distribution de l'acide citrique et ses sels.
La **figure 2** correspond au diagramme de poudre du citrate trisodique monoperhydrate dihydrate de l'exemple 2 (λ = 1,5418 Å).
La **figure 3** représente l'unité asymétrique et l'étiquetage schématique du citrate trisodique monoperhydrate dihydrate de l'exemple 2.
La **figure 4** correspond au diagramme de poudre du citrate disodique perhydrate de l'exemple 3 (λ = 1,5418 Å).
La **figure 5** illustre le diffractogramme de la co-cristallisation d'une mole de citrate disodique perhydrate et de 3 moles d'urée perhydrate (λ = 1,5418 Å), selon l'exemple 4.
La **figure 6** correspond à la concentration logarithmique des différentes souches pathogènes initialement présentes et à la diminution logarithmique des souches après traitement avec la formulation 3 à une concentration en peroxyde d'hydrogène C1 (= 0,5%) et C2 (= 3%).
La **figure 7** correspond à des images microscopiques du champignon *Erysiphe necator.* Figure 7A : dans de l'eau. Figure 7B : en présence de citrate perhydrate (10 mg/ml).
La **figure 8** illustre les résultats obtenus lors de l'évaluation de l'efficacité de la formulation 3 de l'invention en plein champ, pour le traitement de l'oïdium, selon l'exemple 5.5 Le pourcentage d'incidence et de sévérité de la maladie est consigné, que ce soit sur les feuilles (figure 8A) ou sur le raisin (figure 8B).

### EXEMPLES

### Exemple 1 : Synthèse de citrates perhydrates

Un procédé de préparation des citrates perhydrates consiste à cristalliser du peroxyde d'hydrogène avec un sel de l'acide citrique.

Les citrates perhydrates faisant l'objet de la présente invention peuvent être produits à partir du procédé de préparation présenté ci-dessous.

Les équipements susceptibles d'être utilisés consistent en un doseur gravimétrique des matières premières, une extrudeuse bivis pour la réaction de cristallisation en slurry, suivis d'un sécheur à lit fluidisé, ou d'un sécheur micro-onde sous vide, et d'un granulateur.

Les matières premières au procédé sont du peroxyde d'hydrogène, sous la forme d'une solution aqueuse concentrée de 30 à 80%, et un sel de l'acide citrique. Le sel d'acide citrique peut, entre autres, être obtenu par neutralisation partielle ou totale de l'acide citrique avec un carbonate, un hydroxyde ou l'un de ses propres sels, dont les cations sont choisis parmi les métaux alcalins, alcalino-terreux, de transition ou pauvres adéquats à la production du citrate perhydrate souhaité de la présente invention. La neutralisation de l'acide citrique peut, entre autres, être réalisée au préalable de la réaction de cristallisation par un mélange des poudres ou directement au sein de l'extrudeuse par injection individuelle de chaque composant.

### Étape 1 : Réaction de cristallisation

À l'aide d'un doseur gravimétrique, les matières premières sont injectées dans une extrudeuse bivis. La quantité des matières premières injectées respecte la stœchiométrie du citrate perhydrate souhaité de la présente invention. Par exemple, dans le cas de la préparation de citrate disodique monoperhydrate anhydre, un mode de production possible est la cristallisation de 2 moles de citrate trisodique et 1 mole d'acide citrique avec 3 moles de peroxyde d'hydrogène.

Les matières premières se mélangent au sein de l'extrudeuse bivis et commencent à cristalliser à température et temps de séjour contrôlés pour produire un solide en sortie de l'extrudeuse. Par exemple, dans le cas de la production d'un citrate disodique monoperhydrate anhydre, la température de cristallisation est de 55-65°C et le temps de séjour est approximativement 1 minute, lorsque les matières premières solides utilisées sont anhydres. Il est à noter que l'utilisation de matières premières solides hydratées nécessite une extrudeuse munie d'un système de dégazage, diminue fortement le caractère exothermique de la réaction et peut significativement prolonger le temps de séjour dans l'extrudeuse.

À ce stade, le solide obtenu en sortie d'extrusion est thixotropique et peut être aisément mis en forme, tel qu'en granules par exemple, sans s'agglomérer.

Afin de finaliser la cristallisation du citrate perhydrate souhaité de la présente invention, le solide obtenu en sortie de l'extrudeuse est refroidi par convection naturelle jusqu'à 15-25°C. Le rejet de l'excédent d'eau à la réaction de cristallisation est observable visuellement à la surface du solide.

### Étape 2 : Séchage et broyage

Une fois cristallisé, le citrate perhydrate peut être séché à température contrôlée à l'aide d'un sécheur à lit fluidisé ou d'un sécheur micro-onde sous vide, puis broyé à la granulométrie souhaitée étant donné que le produit n'est, à ce stade final, plus thixotropique. Par exemple, dans le cas de la production d'un citrate disodique monoperhydrate anhydre, la température de séchage est de 40-60°C.

### Variante de production d'un citrate perhydrate co-cristallisé avec de l'urée perhydrate.

Un citrate perhydrate selon la présente invention peut être produit par co-cristallisation avec de l'urée pour former un co-cristal de citrate perhydrate et urée perhydrate.

Pour ce faire, de l'urée est également injectée par dosage gravimétrique dans l'extrudeuse bivis. Les ratios molaires à respecter vont de 1 à 5 moles d'urée par mole de sel d'acide citrique, préférentiellement 3 moles d'urée par mole de sel d'acide citrique.

La quantité de peroxyde d'hydrogène est à adapter pour respecter la stochiométrie des cristaux souhaités de citrate perhydrate issu de la présente invention et d'urée perhydrate. Par exemple, dans le cas de la production d'une co-cristallisation d' 1 mole de citrate trisodique monoperhydrate dihydrate et de 3 moles d'urée perhydrate ; 4 moles de peroxyde d'hydrogène cristallisent avec 1 mole de citrate trisodique et 3 moles d'urée.

Cette méthode de production présente des avantages importants en comparaison à une production indépendante d'urée perhydrate que l'on viendrait mélanger sous forme solide aux citrates perhydrates produits. En effet, l'urée perhydrate proposé sur le marché européen est vendu à approximativement 20 €/kg alors que l'urée est, quant à elle, vendue à moins d' 1 €/kg. De plus, l'urée perhydrate produit indépendamment est généralement stabilisé par l'ajout de stabilisant s'avérant plus ou moins toxique, ne le rendant pas compatible avec un biocide biobasé, zéro résidu tel que les citrates perhydrates de la présente invention, alors que l'urée perhydrate produit par co-cristallisation avec un citrate perhydrate est stable sans l'ajout de stabilisants. Cela est probablement la conséquence de la présence de citrate comme stabilisant naturel et non-toxique.

### Variante de production d'un citrate perhydrate à partir d'une solution alcoolique.

Un citrate perhydrate peut être obtenu par cristallisation en solution alcoolique.

Pour ce faire, une première solution est préparée contenant le sel de l'acide citrique et du peroxyde d'hydrogène dont les quantités respectent la stœchiométrique du citrate perhydrate souhaité issu de la présente invention. Le sel d'acide citrique peut, entre autres, être obtenu par neutralisation partielle de l'acide citrique avec un carbonate, un hydroxyde ou l'un de ses propres sels, dont les cations sont choisis parmi les métaux alcalins, alcalino-terreux, de transition ou pauvres adéquats à la production du citrate perhydrate souhaité issu de la présente invention.

Une deuxième solution est préparée : une solution alcoolique, dont l'alcool contient en particulier de 1 à 5 atomes de carbone et qui peut plus particulièrement être l'éthanol. Cette solution peut également être l'alcool lui-même.

Ces deux solutions sont ensuite mélangées. Le ratio volumique de la solution alcoolique se situe entre 3,7 :1 et 8 :1 par rapport à la première solution aqueuse contenant le citrate et le peroxyde d'hydrogène.

Les citrates perhydrates produits précipitent sous forme de cristaux et sont récupérés à l'aide de techniques de séparation liquide/solide connues de l'homme de métier (filtration, centrifugation, etc.) et sont éventuellement séchés à 40-60°C.

### Exemple 2 (comparatif) : Citrate trisodique monoperhydrate dihydrate

Le citrate trisodique monoperhydrate dihydrate a été préparé comme indiqué à l'exemple 1.

### 2.1. Structure cristalline

### Matériel et méthodes

### Diffraction des rayons X sur poudre

Les échantillons ont été délicatement broyés en poudre fine à l'aide d'un pilon et d'un mortier. Les données de diffraction des rayons X sur poudre ont été recueillies à l'aide d'un diffractomètre PANalytical XPERT-PRO (géométrie Bragg-Brentano, rayonnement Cu Kα (λ = 1,5418 Å), réglage du générateur : 45 kV et 30 mA). Les diagrammes de poudres ont été mesurés de 4 à 40° en 2θ et le temps de mesure était de 6 à 15 minutes.

### Résultats

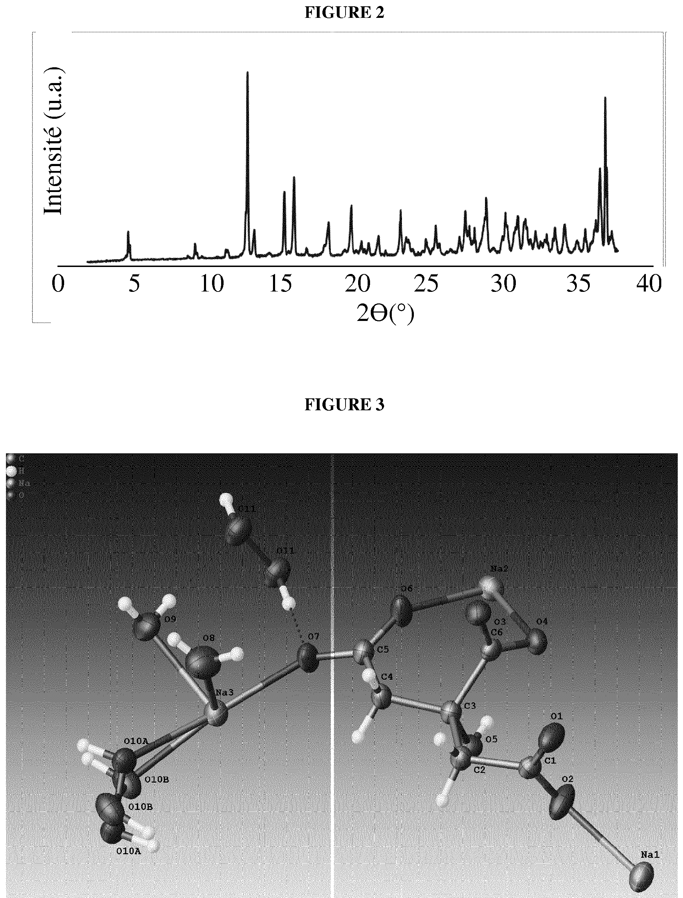

Le diffractogramme obtenu, présenté à la figure 2, ainsi que les raies caractéristiques, au tableau 1, montrent une structure cristalline inconnue, qui diffère du produit de départ, le citrate de tri-sodium, et de toutes ses formes hydratées :

**Tableau 1 : Raies caractéristiques du citrate trisodique monoperhydrate dihydrate**

| **Pics** | **2Θ** | **Intensité relative** | **index hkl** |
|---|---|---|---|
| 1 | 9,924509 | 100 | 0 0 1 |
| 2 | 8,740994 | 5,95 | 0 1 0 et 0 1 -1 |
| 3 | 6,148828 | 0,87 | 1 0 0 |
| 4 | 5,519614 | 15,76 | -1 0 1 |
| 5 | 5,483783 | 36,95 | 0 -1 2 |
| 6 | 5,372527 | 51,54 | -1 1 0 |
| 7 | 4,959513 | 16,42 | -1 -1 1 et 0 0 2 |
| 8 | 4,871902 | 5,55 | 0 -2 1 |
| 9 | 4,740059 | 4,39 | 1 1 0 |
| 10 | 4,413263 | 36,26 | -1 1 1 |
| 11 | 4,381867 | 6,14 | 0 -2 2 |
| 12 | 4,364081 | 19,23 | 0 2 0 |
| 13 | 4,094722 | 2,37 | -1 0 2 |
| 14 | 4,000887 | 38,77 | 1 -2 1 et 1 -1 2 |
| 15 | 3,679708 | 4,92 | 0 -1 3 |
| 16 | 3,660295 | 10,8 | 1 0 2 et -1 -2 1 |
| 17 | 3,62327 | 22,99 | 1 -2 2 |
| 18 | 3,514807 | 11,02 | -1 -2 2 |
| 19 | 3,483591 | 3,3 | 0 -2 3 |
| 20 | 3,466081 | 14,3 | 0 2 1 |

Les caractéristiques du cristal de citrate trisodique monoperhydrate dihydrate sont consignées dans le tableau 2 suivant :

**Tableau 2 : Caractéristiques de la structure cristalline du citrate trisodique monoperhydrate dihydrate**

| | | |
|---|---|---|
| Formule chimique | | C6 H11 O11 Na3 |
| Poids moléculaire | | 328,12 g/mol |
| Citrate de sodium | % massique | 78,7 |
| | ratio molaire | 1 |
| Peroxyde d'hydrogène | % massique | 10,4 |
| | ratio molaire | 1 |
| Eau | % massique | 10,9 |
| | ratio molaire | 2 |
| Groupe d'espace | | P-1 |
| Système cristallin | | Triclinique |
| Longueur de maille (a) | | 6,2400 (4) Å |
| Longueur de maille (b) | | 9,8204(5) Å |
| Longueur de maille (c) | | 11,1233(7) Å |
| α | | 115,846(6) Å |
| β | | 93,600(5) Å |
| γ | | 95,350(5) Å |
| Volume de maille | | 606,69(7) Å |
| ρ calculé | | 1,796 cm³ |

L'unité asymétrique contient un anion citrate totalement déprotonné, trois cations de sodium, deux molécules d'eau et deux demi-molécules de peroxyde d'hydrogène. Ainsi, la formule chimique donnée est *Nα*₃*C*₆*H*₁₁*O*₁₁, comme présentée ci-dessous et à la figure 3.

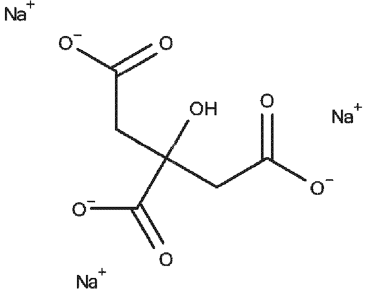

HO - OH

H₂O

H₂O

### Structure moléculaire du citrate trisodique monoperhydrate dihydrate

### 2.2 Hydrosolubilité

La solubilité des composés de l'invention est déterminée par des expériences à concentrations croissantes en composé de l'invention dans l'eau désionisée. Les résultats montrent que le citrate trisodique monoperhydrate dihydrate est totalement soluble dans l'eau ( > 900 g/l à 20°C).

### 2.3. pH d'une solution aqueuse du composé

Les mesures de pH (à l'aide d'un pH-mètre) montrent qu'une solution aqueuse du citrate trisodique monoperhydrate dihydrate dilué 20x est de 7,6 (± 0,3).

### Exemple 3 (invention) : Citrate disodique monoperhydrate anhydre

Le citrate disodique monoperhydrate anhydre a été préparé comme indiqué à l'exemple 1.

### 3.1. Structure cristalline

Les données de diffraction des rayons X sur poudre ont été recueillies à l'aide d'un diffractomètre Cu Kα comme décrit plus haut et complétées par des mesures avec rayonnement synchrotron.

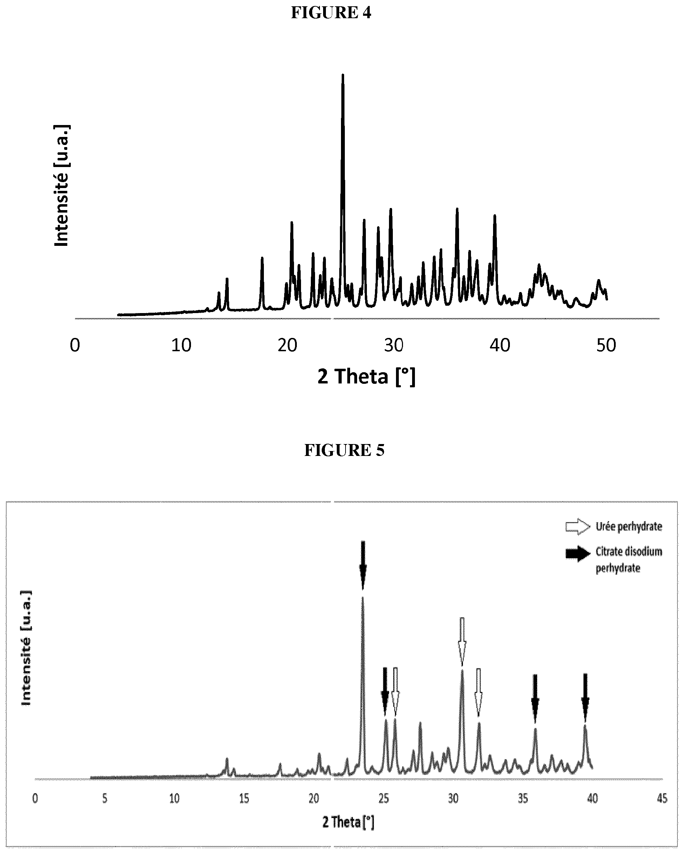

Le diffractogramme obtenu, présenté à la figure 4, et ses raies caractéristiques, consignées dans le tableau 3, montre une structure cristalline inconnue des bases de données, qui diffère de l'acide citrique, du citrate trisodique et de toutes ses formes hydratées, du citrate de disodium et de toutes ses formes hydratées, ainsi que du citrate trisodique perhydrate.

**Tableau 3 : Raies caractéristiques du citrate disodique monoperhydrate anhydre**

| **Pics** | **2Θ** | **Intensité relative** | **Index hkl** |
|---|---|---|---|
| 1 | 7,10442 | 2,42 | 0 1 1 |
| 2 | 6,53808 | 6,93 | 1 1 1 |
| 3 | 6,19763 | 11,5 | 0 2 0 |
| 4 | 5,03008 | 18,94 | 2 2 0 |
| 5 | 4,83206 | 0,76 | 1 2 1 |
| 6 | 4,45527 | 10,07 | 3 1 1 |
| 7 | 4,34762 | 32,9 | 2 2 1 |
| 8 | 4,29193 | 11,19 | 4 0 0 |
| 9 | 4,21333 | 17,21 | 3 2 0 |
| 10 | 3,97003 | 19,47 | 1 1 2 |
| 11 | 3,85293 | 13,47 | 4 0 1 et 2 0 2 |
| 12 | 3,78756 | 19,04 | 3 2 1 |
| 13 | 3,67804 | 12,75 | 4 1 1 |
| 14 | 3,53769 | 100 | 4 2 0 |
| 15 | 3,47303 | 5,61 | 1 2 2 |
| 16 | 3,42705 | 7,44 | 2 3 1 |
| 17 | 3,32368 | 6,66 | 3 1 2 |
| 18 | 3,2801 | 32,86 | 2 2 2 |
| 19 | 3,13095 | 31,11 | 3 3 1 |
| 20 | 3,09689 | 19,5 | 5 1 1 |

Les caractéristiques propres au cristal de citrate disodique perhydrate sont consignées dans le tableau 4.

**Tableau 4 : Caractéristiques de la structure cristalline du citrate disodique monoperhydrate anhydre**

| Système cristallin | Orthorhombique # 61 *Pbca* |
|---|---|
| Longueur de maille (a) | 8.6396(25) Å |
| Longueur de maille (b) | 12,433(4) Å |
| Longueur de maille (c) | 17,199(5) Å |
| Volume de maille | 1847,5(8) Å³ |

Les mesures concernant les liaisons H sont consignées dans le tableau 5 ci-après :

**Tableau 5 : Liaisons hydrogènes dans la structure cristalline du citrate disodique monoperhydrate anhydre (*intramoléculaire)**

| Liaison H | D-A, Å | H···A, Å | D ... A, Å | D-H ... A, Å | Recouvrement, *e* | E, kcal/m |
|---|---|---|---|---|---|---|
| O12-H21···O13 | 1,041 | 1,507 | 2,546 | 175,0 | 0,092 | 16,6 |
| 017-H18···O22 | 0.976 | 1,852 | 2,770 | 155,7 | 0,041 | 11,1 |
| 017-H18···O13 | 0.976 | 2,557* | 3,063 | 112,5 | 0,009 | 5,2 |
| O22-H24···O13 | 1.015 | 1,585 | 2,598 | 174,7 | 0,080 | 15,5 |
| O23-H25···O13 | 0.994 | 1,605 | 2,596 | 174,8 | 0,064 | 13,8 |
| C4-H10···O13 | 1.094 | 2,340 | 3,270 | 141,8 | 0,015 | |
| C2-H7···O13 | 1.095 | 2,482* | 3,199 | 121,9 | 0,012 | |

L'unité asymétrique correspond à la formule Na₂HC₆H₅O₇(H₂O₂), et contient ainsi un anion citrate, deux cations de sodium et une molécule de peroxyde d'hydrogène, comme présentée ci-dessous. Il n'y a pas de vides dans la structure pour accommoder une molécule d'eau.

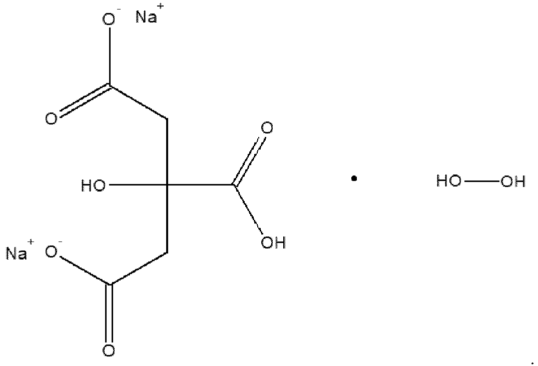

### 3.2. Hydrosolubilité

Le citrate disodique perhydrate est totalement soluble dans l'eau ( > 800 g/l à 20°C).

### 3.3. pH d'une solution aqueuse du composé

Les mesures de pH (à l'aide d'un pH-mètre) montrent que dilué (20x) dans l'eau, les cristaux citrate de disodique perhydrate obtenus ont un pH de 5,2 (± 0,3).

### 3.4. Analyse thermogravimétrique (TGA)

Des analyses thermogravimétriques d'une poudre cristalline de citrate perhydrate disodique selon l'exemple 1 ont été réalisées, pour évaluer son comportement à une température de 100 à 220°C.

Les courbes de perte de masse en TGA ont été obtenues avec un instrument TA instruments (Waters) sous une atmosphère d'azote et dans la gamme de température comprises entre 30 et 250°C. Une rampe de température de 10°C/min a été imposée, suivie d'une isotherme à la température finale (250°C dans le cadre de cette analyse) pendant une période de 10 minutes. Une perte de masse de moins de 1 % a lieu en dessous de 140°C, correspondant probablement à une perte d'eau de cristallisation. La poudre a ensuite perdu entre 2 et 3 % de sa masse entre 140 et 220 °C.

Ces résultats montrent une excellente stabilité aux hautes températures.

### Exemple 4 : Co-cristallisation d'une mole de citrate disodique monoperhydrate anhydre et de 3 moles d'urée perhydrate

### 4.1. Structure cristalline

Le diffractogramme obtenu est présenté à la figure 5.

Le spectre de diffraction obtenu montre que la poudre produite est une co-cristallisation de citrate disodique monoperhydrate anhydre et de l'urée perhydrate, dont les pics les plus caractéristiques sont mis en évidence.

### 4.2 pH d'une solution aqueuse du composé

Le pH d'une solution aqueuse des co-cristaux d'une mole de citrate disodique monoperhydrate anhydre et de 3 moles d'urée perhydrate dilués 20x est de 5,2 (± 0,3).

### Exemple 5 : Performances

### 1. Formulation 1 à base du citrate trisodique monoperhydrate dihydrate et d'urée perhydrate co-cristallisés (comparatif)

### 1.1. Préparation de la formulation

200 g d'une co-cristallisation contenant 1 mole de citrate trisodique monoperhydrate dihydrate et 4 moles d'urée perhydrate sont mélangés avec 21 g d'urée et 55 g d'acide citrique anhydre. Le mélange de poudres est ensuite dissous dans de l'eau désionisée à différentes concentrations.

### 1.2. Performances biocides sur biotests

La formulation 1 est testée en biotest sur divers pathogènes. Les résultats sont présentés dans le tableau 6.

**Tableau 6 : Concentration efficace [mg/ml] de la formulation 1.**

| | |
|---|---|
| *Plasmopara viticola* | 24 |
| *Botrytis cinerea* | <5 |
| *Guignardia bidwellii* | 5 |
| *Helminthosporium solani* | 25 |
| *Collelotrichum coccodes* | 25 |
| *Monilia laxa souche 623* | 18 |
| *Monilia laxa souche INRA* | 50 |
| *Serpula lacrimens* | 18 |
| *Staphylococcus aureus* | 0,064 |
| *Pseudomonas aeruginosa* | 0,256 |
| *Erwinia amylovora* | 1,024 |
| *Ralstonia souche 06* | 0,512 |
| *Ralstonia souche R1* | 0,512 |

### 2. Formulation 2 à base du citrate disodique monoperhydrate anhydre

### 2.1. Préparation de la formulation

La poudre de l'exemple 3 est dissoute dans de l'eau désionisée à différentes concentrations.

### 2.2. Performances antifongiques

La formulation 2 est testée en biotest sur divers pathogènes. Les résultats sont présentés dans le tableau 7.

**Tableau 7 : Concentration efficace [mg/ml] de la formulation 2.**

| | |
|---|---|
| *Erysiphe necator* | 10 |
| *Botrytis cinerea* | 5-10 |
| *Monilia laxa souche 623* | 10-20 |
| *Monilia laxa souche INRA* | 34-50 |
| *Monilia fructigena* | 34-50 |

### 3. Formulation 3 à base du citrate disodique monoperhydrate anhydre et d'urée perhydrate co-cristallisés

### 3.1. Préparation de la formulation

La poudre de l'exemple 4 est dissoute dans de l'eau désionisée à différentes concentrations.

### 3.2. Performances antifongiques

La formulation 3 est testée en biotest sur les champignons *Plasmopara viticola, Erysiphe necator, Guignardia bidwellii et Monilia fructigena.* Le résultat est présenté au tableau 8.

**Tableau 8 : Concentration efficace [mg/ml] de la formulation 3.**

| | |
|---|---|
| *Plasmopara viticola* | 30 |
| *Erysiphe necator* | 10 |
| *Botrytis cinerea* | 1-5 |
| *Guignardia bidwellii* | 25-50 |
| *Monilia laxa souche 623* | 10-20 |
| *Monilia laxa souche INRA* | 17-25 |
| *Monilia fructigena* | 17-25 |

### 4. Formulation 4 à base du citrate disodique monoperhydrate anhydre et d'urée perhydrate co-cristallisés

### 4.1. Préparation de la formulation

62 g de la poudre décrite à l'exemple 4 sont mélangés avec 10 g d'acide citrique anhydre, 3 g d'acide lactique, 2 g de lactate de calcium anhydre, 1,5 g d'un agent tensioactif et 1,5 g d'un agent desséchant. Le mélange de poudre est ensuite dissous dans de l'eau désionisée à différentes concentrations.

### 4.2. Performances antibactériennes

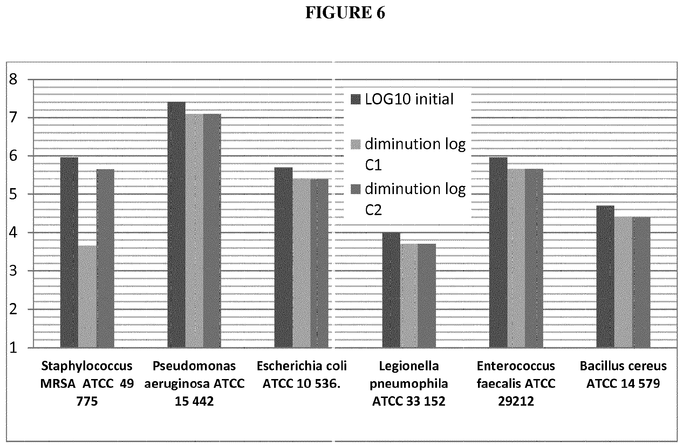

La formulation 4 est testée en biotest sur six souches pathogènes selon la norme EN1040. Les résultats sont présentés à la figure 6.

### 5. Essais in vitro

L'efficacité des formulations 2 et 3 telles que décrites plus haut vis-à-vis à l'aide de biotests sur le champignon *Botritys cinerea* sur la germination des conidies (Tableau 9) et le développement du mycélium (Tableau 10) en comparaison avec le fongicide chimique de référence (Teldor, Bayer), ainsi que des biotests sur le champignon *Venturia inaequalis* sur la germination des conidies (Tableau 11) en comparaison avec le fongicide chimique de référence (Merpan, Adam France).

**Tableau 9 : efficacité (%) des formulations 2 et 3 vis-à-vis de Botritys cinerea (germination des conidies)**

| **formulation/ concentration (mg/ml)** | **2** | **5** | **10** |
|---|---|---|---|
| Formulation 2 | - | 100 | 100 |
| Formulation 3 | 100 | 100 | 100 |
| Teldor (référence) | 79 | 81 | 81 |

**Tableau 10: efficacité (%) des formulations 2 et 3 vis-à-vis de Botritys cinerea (développement du mycélium)**

| **formulation/ concentration (mg/ml)** | **5** | **10** | **15** | **20** | **30** |
|---|---|---|---|---|---|
| Formulation 2 | 25-49,99 | 50-74,999 | 50-74,999 | 50-74,999 | 75-100 |
| Formulation 3 | 75-100 | 75-100 | 75-100 | 75-100 | 75-100 |
| Teldor (référence) | 50-74,999 | 50-74,999 | 50-74,999 | 50-74,999 | - |

**Tableau 11 : efficacité (%) des formulations 2 et 3 vis-à-vis de du champignon Venturia inaequalis (germination des conidies)**

| **Formulation / concentration (mg/ml)** | **2** | **5** | **10** |
|---|---|---|---|
| Formulation 2 | 100 | 100 | 100 |
| Formulation 3 | 100 | 100 | 100 |
| Merpan (référence) | 100 | 100 | 100 |

### 6. Performances antifongiques : autre validation plein champs

L'expérience est réalisée dans un vignoble suisse conformément aux normes de Bonne Pratiques Agricole (BPE) telles que définies à l'article R 253-1 du Code Rural (FR) pour les données relatives à l'évaluation biologique des produits phytosanitaires. L'essai vise à évaluer le développement de l'oïdium à la fois sur les feuilles et les grappes. Les plants sont traités hebdomadairement par application d'une bouillie de 200 l/ha. Les parcelles d'essais sont décrites comme indiqué ci-dessous :
- Une parcelle non traitée (TNT) ;
- Une parcelle traitée avec la formulation 3 à 30g/l, à laquelle a été ajouté 1% de SiO₂ et 0,5 % d'heptaméthyltrisiloxanes (agent mouillant, De Sangosse - Agridyne), ce qui correspond à 6 kg par hectare de vigne (Biogel solo);
- Une parcelle traitée avec un fongicide chimique conventionnel de référence (programme conventionnel);
- Une parcelle traitée avec un fongicide de référence autorisé en agriculture biologique (programme bio).

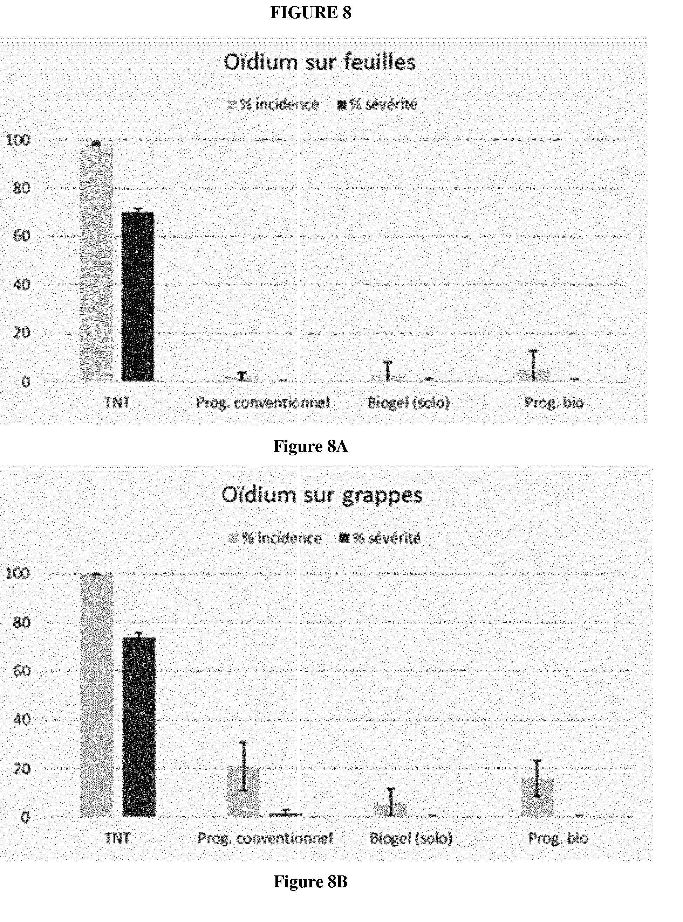

L'évaluation de l'efficacité de la formulation testée est réalisée à partir de 2 critères ; le pourcentage d'incidence et de sévérité de la maladie, que ce soit sur les feuilles (figure 8A) ou sur le raisin (figure 8B).

### Exemple 6 : Instabilité des solutions concentrées d'acide citrique et de peroxyde d'hydrogène

La stabilité d'une solution concentrée en peroxyde d'hydrogène et acide citrique est étudiée.

Pour ce faire, une solution contenant 37,5% de peroxyde d'hydrogène et 25% d'acide citrique est préparée. La concentration en peroxyde d'hydrogène est ensuite mesurée à partir d'un titrage permanganométrique à trois reprises sur deux mois. La teneur en peracides est également mesurée à partir d'une méthode colorimétrique sur bandelettes.

Les résultats sont présentés dans le tableau 12 :

**Tableau 12 : Résultats de l'évolution d'une solution contenant 37,5% en peroxyde d'hydrogène et 25% d'acide citrique**

| **Date des mesures** | **%w. Peroxyde d'hydrogène** | **%w. Peracides** |
|---|---|---|
| J0 | 37,5 | 0 |
| J0+ 16 jours | 32,6 | 5 |
| J0+ 2 mois | 24,6 | 5 |

Ces résultats montrent que la solution perd 34,4% de sa concentration en peroxyde d'hydrogène après 2 mois. On voit également apparaître 5% de peracides en solution après 18 jours. De plus, une augmentation de la pression suivie d'un dégagement gazeux est observée au fil du temps.

### Exemple 7 : Performances comparatives des citrates perhydrates de l'invention face à des solutions aqueuses de peroxyde d'hydrogène, de citrates et acide citrique

### 1. Action sur Botrytis cinerea

Des essais réalisés selon un protocole standard sur différentes cibles fongiques, dont *Botrytis cinerea,* ont montré qu'à concentrations équivalentes, ni le citrate seul, ni le peroxyde d'hydrogène seul n'ont d'effet fongicide ou fongistatique, au contraire du composé de l'invention (cf. tableau 13 ci-après).

Ceci est d'ailleurs confirmé par Gil-ad et al. (FEMS Microbiology Letters 1999, 176, 455-461), indiquant que *Botrytis cinerea* peut germer en présence de peroxyde d'hydrogène à des concentrations allant jusqu'à 180 mM (6 mg/mL), et son mycélium peut se développer à des concentrations encore supérieures.

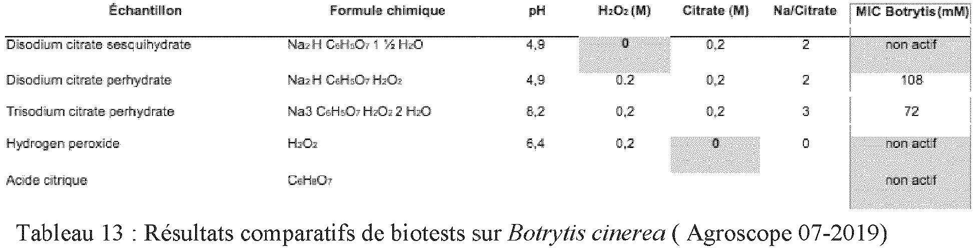

Le citrate perhydrate disodique cristallise de préférence avec le peroxyde d'hydrogène, plutôt qu'avec de l'eau et forme une barrière biocide réactive. En effet, lorsque le citrate perhydrate disodique est mis en solution aqueuse pour pulvérisation, c'est-à-dire que le citrate de disodium et le peroxyde d'hydrogène sont pulvérisés sur une surface, l'eau s'évapore et le citrate perhydrate disodique se cristallise à nouveau pour former une barrière biocide réactive alors qu'une solution de peroxyde d'hydrogène ne fait que s'évaporer. Ceci permet un effet persistant du produit de l'invention.

### 2. Action sur Staphylococcus Aureus

Des essais ont été réalisés selon un protocole standard sur *Staphylococcus Aureus MRSA,* comparant le citrate disodique perhydrate de l'invention au sodium citrate, au peroxyde d'hydrogène, et à un antiseptique de référence.

Les résultats de ces essais sont consignés dans le tableau 14 :

**Tableau 14 : Résultats comparatifs de biotests sur Staphylococcus Aureus MRSA**

| | **Valeur de MIC** | **%** | **mM** |
|---|---|---|---|
| Citrate de sodium (pH 5->8) | 3,20 mg/ml | 0,320 | 12,40 -> 13,56 |
| Peroxyde d'hydrogène | 0,94 mg/ml | 0,094 | 27,58 |
| PVPI (Povidone iodée) | 6,25 mg/ml | 0,625 | 17,13 |
| Ethanol | 87,5 mg/ml | 8,750 | 1899,35 |
| **Citrate disodique perhydrate de l'invention** | **0,03 mg/ml** | **0,003** | **0,12** |

Vu la structure moléculaire du citrate disodique perhydrate, la comparaison de l'efficacité du composé de l'invention vis-à-vis du citrate de sodium et du peroxyde d'hydrogène est aisée (en équimolaire).

Il est à remarquer que le citrate disodique perhydrate a une efficacité inhibitrice du développement (MIC) de *Staphylococcus aureus MRSA* 230 fois supérieure à celle du peroxyde d'hydrogène seul et 103 fois supérieure au citrate de sodium.

## Revendications

1. Citrate perhydrate de métal alcalin, qui est un citrate disodique monoperhydrate, de préférence un citrate disodique monoperhydrate anhydre.

2. Citrate perhydrate de métal alcalin selon la revendication 1, sous forme de poudre, la poudre étant stable physiquement et chimiquement sans modification significative de son état physique ou de sa composition chimique après un an à une température de 20 à 25°C, notamment de sa concentration en oxygène actif.

3. Citrate perhydrate de métal alcalin selon l'une quelconque des revendications 1 à 2, soluble dans l'eau, en particulier présentant une solubilité supérieure ou égale à 850 g/l à 25°C dans l'eau.

4. Citrate perhydrate de métal alcalin selon l'une quelconque des revendications précédentes, présentant en solution aqueuse un pH compris entre 4 et 8.

5. Citrate perhydrate de métal alcalin selon l'une quelconque des revendications 1 à 3, sous la forme d'un cristal constitué de citrate disodique et de peroxyde d'hydrogène.

6. Composition comprenant un citrate perhydrate tel que défini dans l'une quelconque des revendications 1 à 2 ou 5, et de l'urée perhydrate, notamment sous la forme d'un co-cristal urée-peroxyde d'hydrogène, ladite composition comprena nt en particulier des cristaux de citrate disodique perhydrate et des cristaux d'urée perhydrate, notamment sous la forme d'un co-cristal urée-peroxyde d'hydrogène.

7. Composition pharmaceutique ou phytopharmaceutique constitué de ou comprenant un citrate disodique perhydrate selon l'une quelconque des revendications 1 à 5.

8. Utilisation non-thérapeutique d'un citrate disodique perhydrate selon l'une quelconques des revendications 1 à 5 en tant que biocide.

9. Utilisation selon la revendication 8, dans laquelle ledit citrate disodique perhydrate est utilisé en présence d'eau et/ou en présence d'au moins un composé additionnel choisi parmi les agents régulateurs de pH, les agents anti-agglomérants, les tensioactifs, les agents mouillants, les agents anti-mousse, les anti-dérives, les épaississants, les agents moussants, les agents de solidification, les engrais, les produits phytopharmaceutiques, les stabilisants et leurs mélanges, le composé additionnel étant notamment choisi parmi les glycolipides.

10. Utilisation selon la revendication 8 ou 9, pour inhiber ou prévenir la croissance d'un pathogène sur ou dans une plante, ledit citrate étant en particulier appliqué à la surface de la plante, plus particulièrement à hauteur de 25 à 1000 ng/dm², où le pathogène est de préférence sélectionné parmi les virus, les bactéries, les champignons et les pseudo-champignons et où la plante est de préférence choisie parmi les plantes produisant des fruits, les plantes produisant des légumes, les plantes ornementales, le gazon et les céréales.

11. Utilisation selon l'une quelconque des revendications 8 à 12 pour désinfecter un fluide ou une surface, en particulier l'eau, l'air, les sols, les piscines, les surfaces de travail, les toilettes.

12. Procédé de préparation d'un citrate perhydrate de métal alcalin selon l'une quelconque des revendications 1 à 5, ledit procédé comprenant :
(i) une étape de mise en contact d'un citrate disodique avec du peroxyde d'hydrogène, pour obtenir ledit citrate disodique perhydrate, ladite étape (i) étant éventuellement précédée d'une étape de neutralisation d'un acide citrique par un hydroxyde, un carbonate ou un citrate de sodium, afin d'obtenir le citrate disodique tel que mentionné dans l'étape (i), ou (i') une étape de mise en contact d'acide citrique ; d'un hydroxyde, carbonate ou citrate de sodium ; et de peroxyde d'hydrogène, pour obtenir ledit citrate disodique perhydrate.

13. Procédé de préparation d'une composition selon la revendication 6, ledit procédé comprenant une étape (a) de co-cristallisation par mise en contact d'un citrate disodique ; d'urée ; et de peroxyde d'hydrogène. ladite étape (a) étant éventuellement précédée d'une étape de neutralisation d'un acide citrique par un hydroxyde, un carbonate ou un citrate de sodium, afin d'obtenir le citrate disodique tel que mentionné dans l'étape (a), ou (a') une étape de mise en contact d'acide citrique ; d'un hydroxyde, carbonate ou citrate de sodium ; d'urée ; et de peroxyde d'hydrogène, pour obtenir ledit citrate disodique perhydrate.

## Patentansprüche

1. Citratperhydrat eines Alkalimetalls, bei dem es sich um ein Dinatriumcitrat-Monoperhydrat, vorzugsweise um ein wasserfreies Dinatriumcitrat-Monoperhydrat, handelt.

2. Citratperhydrat eines Alkalimetalls nach Anspruch 1, in Pulverform, wobei das Pulver physikalisch und chemisch stabil ist ohne signifikante Veränderung seines physikalischen Zustands oder seiner chemischen Zusammensetzung nach einem Jahr bei einer Temperatur von 20 bis 25 °C, besonders seines Gehalts an aktivem Sauerstoff.

3. Citratperhydrat eines Alkalimetalls nach einem der Ansprüche 1 bis 2, wasserlöslich, insbesondere mit einer Löslichkeit größer oder gleich 850 g/l bei 25 °C in Wasser.

4. Citratperhydrat eines Alkalimetalls nach einem der vorstehenden Ansprüche, das in wässriger Lösung einen pH-Wert zwischen 4 und 8 aufweist.

5. Citratperhydrat eines Alkalimetalls nach einem der Ansprüche 1 bis 3, in Form eines Kristalls, bestehend aus Dinatriumcitrat und Wasserstoffperoxid.

6. Zusammensetzung, umfassend ein wie in einem der Ansprüche 1 bis 2 oder 5 definiertes Perhydratcitrat sowie Harnstoffperhydrat, besonders in Form eines Harnstoff-Wasserstoffperoxid-Co-Kristalls, wobei die Zusammensetzung insbesondere Kristalle eines Dinatriumcitrat-Perhydrats und Kristalls eines Harnstoffperhydrats, besonders in Form eines Harnstoff-Wasserstoffperoxid-Co-Kristalls, umfasst.

7. Zur Verwendung in der Pharmazie oder im Pflanzenschutz bestimmte Zusammensetzung, bestehend aus oder umfassend ein Dinatriumcitrat-Perhydrat nach einem der Ansprüche 1 bis 5.

8. Nicht-therapeutische Verwendung eines Dinatriumcitrat-Perhydrats nach einem der Ansprüche 1 bis 5 als Biozid.

9. Verwendung nach Anspruch 8, bei der das Dinatriumcitrat-Perhydrat in Gegenwart von Wasser und/oder in Gegenwart mindestens eines weiteren Zusatzstoffs, ausgewählt aus pH-Reglern, Antiklumpmitteln, Tensiden, Netzmitteln, Antischaummitteln, Antidriftmitteln, Verdickungsmitteln, Schaummitteln, Verfestigungsmitteln, Düngemitteln, Pflanzenschutzmitteln, Stabilisatoren und deren Mischungen, verwendet wird, wobei der Zusatzstoff besonders aus Glykolipiden ausgewählt ist.

10. Verwendung nach Anspruch 8 oder 9, um das Wachstum eines Pathogens auf oder in einer Pflanze zu hemmen oder zu verhindern, wobei das Citrat insbesondere auf der Oberfläche der Pflanze appliziert wird, ganz besonders in einer Menge von 25 bis 1000 ng/dm², wobei das Pathogen vorzugsweise aus Viren, Bakterien, Pilzen und Scheinpilzen ausgewählt ist und wobei die Pflanze vorzugsweise aus Obstpflanzen, Gemüsepflanzen, Zierpflanzen, Rasengras und Getreide ausgewählt ist.

11. Verwendung nach einem der Ansprüche 8 bis 12 zur Desinfektion eines Fluids oder einer Oberfläche, insbesondere von Wasser, Luft, Böden, Schwimmbecken, Arbeitsflächen oder Toiletten.

12. Verfahren zur Herstellung eines Citratperhydrat eines Alkalimetalls nach einem der Ansprüche 1 bis 5, wobei das Verfahren umfasst:
(i) einen Schritt des Kontaktierens von Dinatriumcitrat mit Wasserstoffperoxid zur Gewinnung des Dinatriumcitrat-Perhydrats, wobei dem Schritt (i) gegebenenfalls ein Schritt der Neutralisierung von Zitronensäure mit Natriumhydroxid, Natriumcarbonat oder Natriumcitrat zur Gewinnung des in Schritt (i) genannten Dinatriumcitrats vorausgeht, oder (i') einen Schritt des Kontaktierens von Zitronensäure; Natriumhydroxid, Natriumcarbonat oder Natriumcitrat; und Wasserstoffperoxid zur Gewinnung des Dinatriumcitrat-Perhydrats.

13. Verfahren zur Herstellung einer Zusammensetzung nach Anspruch 6, wobei das Verfahren umfasst: einen Schritt (a) der Co-Kristallisation durch Kontaktieren von Dinatriumcitrat, Harnstoff und Wasserstoffperoxid, wobei dem Schritt (a) gegebenenfalls ein Schritt der Neutralisierung von Zitronensäure mit Natriumhydroxid, Natriumcarbonat oder Natriumcitrat zur Gewinnung des in Schritt (a) genannten Dinatriumcitrats vorausgeht, oder (a') einen Schritt des Kontaktierens von Zitronensäure; Natriumhydroxid, Natriumcarbonat oder Natriumcitrat; Harnstoff; und Wasserstoffperoxid zur Gewinnung des Dinatriumcitrat-Perhydrats.

## Claims

1. An alkali metal citrate perhydrate, which is a disodium citrate monoperhydrate, preferably an anhydrous disodium citrate monoperhydrate.

2. The alkali metal citrate perhydrate according to claim 1, in powder form, the powder being physically and chemically stable without significant change in its physical state or chemical composition after one year at a temperature of 20 to 25°C, in particular its active oxygen concentration.

3. The alkali metal citrate perhydrate according to any one of claims 1 to 2, soluble in water, in particular having a solubility greater than or equal to 850 g/l at 25°C in water.

4. The alkali metal citrate perhydrate according to any one of the preceding claims, having a pH between 4 and 8 in aqueous solution.

5. The alkali metal citrate perhydrate according to any one of claims 1 to 3, in the form of a crystal consisting of disodium citrate and hydrogen peroxide.

6. A composition comprising a perhydrate citrate as defined in any one of claims 1 to 2 or 5, and perhydrate urea, in particular in the form of a urea-hydrogen peroxide co-crystal, said composition comprising in particular crystals of disodium citrate perhydrate and crystals of urea perhydrate, in particular in the form of a urea-hydrogen peroxide co-crystal.

7. A pharmaceutical or phytopharmaceutical composition consisting of or comprising disodium citrate perhydrate according to any one of claims 1 to 5.

8. A non-therapeutic use of disodium citrate perhydrate according to any one of claims 1 to 5 as a biocide.

9. The use according to claim 8, wherein said disodium citrate perhydrate is used in the presence of water and/or in the presence of at least one additional compound selected from pH regulators, anti-caking agents, surfactants, wetting agents, anti-foaming agents, anti-drift agents, thickening agents, foaming agents, solidifying agents, fertilizers, phytopharmaceutical products, stabilizers, and mixtures thereof, the additional compound being selected in particular from glycolipids.

10. The use according to claim 8 or 9, to inhibit or prevent the growth of a pathogen on or in a plant, said citrate being in particular applied to the surface of the plant, more particularly at a level of 25 to 1000 ng/dm², where the pathogen is preferably selected from viruses, bacteria, fungi, and pseudo-fungi, and wherein the plant is preferably selected from fruit-producing plants, vegetable-producing plants, ornamental plants, turf, and cereals.

11. The use according to any one of claims 8 to 12 for disinfecting a fluid or surface, in particular water, air, soils, swimming pools, work surfaces, toilets.

12. A method for preparing an alkali metal citrate perhydrate according to any one of claims 1 to 5, said method comprising:
(i) a step of contacting disodium citrate with hydrogen peroxide to obtain said disodium citrate perhydrate, said step (i) optionally being preceded by a step of neutralizing citric acid with a sodium hydroxide, carbonate or citrate to obtain the disodium citrate as mentioned in step (i), or (i') a step of contacting citric acid; a sodium hydroxide, carbonate or citrate; and hydrogen peroxide, to obtain said disodium citrate perhydrate.

13. The method for preparing a composition according to claim 6, said method comprising a step (a) of co-crystallization by contacting disodium citrate; urea; and hydrogen peroxide, said step (a) optionally being preceded by a step of neutralizing citric acid with a sodium hydroxide, carbonate or citrate, to obtain the disodium citrate as mentioned in step (a), or (a') a step of contacting citric acid; a sodium hydroxide, carbonate or citrate; urea; and hydrogen peroxide, to obtain said disodium citrate perhydrate.
